# EUROPÄISCHE PATENTANMELDUNG

(11) **EP 2 905 614 A1**
(43) Veröffentlichungstag der Anmeldung: **12.08.2015**
(21) Anmeldenummer: 14154607.7
(22) Anmeldetag: 11.02.2014
(51) Int. Cl.: G01N 33/49, G01N 33/68

(54) **Verfahren zur Bestimmung der Aktivität des Blutgerinnungssystems**

(71) Anmelder: Siemens Healthcare Diagnostics Products GmbH, 35041 Marburg (DE)
(72) Erfinder: Althaus, Harald, 35083 Wetter (DE); Schwarz, Herbert, 35102 Lohra (DE); Vitzthum, Frank, New City, NY 10956 (US); Kappel, Andreas, 61479 Glashütten (DE)

(57) **Zusammenfassung**

Die vorliegende Erfindung liegt auf dem Gebiet der Gerinnungsdiagnostik und betrifft ein Verfahren zur Bestimmung der Aktivität des Blutgerinnungssystems, wobei die Menge von Fibrinopeptid A und/oder Fibrinopeptid B in einer Probe bestimmt wird, vorzugsweise mittels Massenspektrometrie.

## Beschreibung

Die vorliegende Erfindung liegt auf dem Gebiet der Gerinnungsdiagnostik und betrifft ein Verfahren zur Bestimmung der globalen Aktivität der Blutgerinnungssystems, wobei die Menge von Fibrinopeptid A und/oder Fibrinopeptid B in einer Probe bestimmt wird, vorzugsweise mittels Massenspektrometrie.

In der Gerinnungsdiagnostik werden sogenannte Globalteste zur Untersuchung der Funktionalität bzw. Aktivität des Blutgerinnungssystems oder des Fibrinolysesystems und sogenannte Einzelteste zur Bestimmung der Aktivität einzelner Blutgerinnungs- oder Fibrinolysefaktoren unterschieden. Die sogenannten Globalteste (z.B. PT oder APTT) erfassen mehrere enzymatische Reaktionen innerhalb bestimmter Bereiche des Blutgerinnungsystems. Sowohl für die Globalteste als auch für die Einzelteste sind unterschiedliche Testformate bekannt. In Bezug auf das Testformat werden im wesentlichen Gerinnungsteste und chromogene Teste unterschieden.

Bei einem Gerinnungstest wird die zu untersuchende Patientenprobe, die üblicherweise aus Plasma besteht, mit einem Gerinnungsaktivator vermischt, der den Gerinnungsprozess startet. Der Gerinnungsprozess führt zur Bildung eines Fibringerinnsels, das mit Hilfe photometrischer Verfahren gemessen werden kann. Die Fibrinbildungsgeschwindigkeit ist ein Maß für die Funktionalität bzw. Aktivität der Blutgerinnungskaskade. Zur Bestimmung der Aktivität eines einzelnen Gerinnungsfaktors in einem Gerinnungstest wird die zu untersuchende Patientenprobe zusätzlich mit einem Mangelplasma vermischt, welches alle Komponenten der Blutgerinnungskaskade supplementiert außer den zu testenden Blutgerinnungsfaktor.

Typische Beispiele für derartige Gerinnungsteste sind die Prothrombinzeit (PT), die auch Quick-Test oder Thromboplastinzeit genannt wird, die aktivierte partielle Thromboplastinzeit (APTT), die Thrombinzeit (TT), die Batroxobinzeit (BT) oder die Ecarinzeit (ECT). Diese Teste und ihre Varianten werden gewöhnlich zum Screening auf Defekte in einem Teilbereich des Gerinnungssystems verwendet (Screeningteste, Globalteste, Suchteste) oder zur Aktivitätsmessung von Einzelfaktoren. Zu den Defekten des Gerinnungssystems, die eine Blutungsneigung oder eine Thromboseneigung zur Folge haben können, gehören zum Beispiel (a) sehr geringe oder sehr hohe Konzentrationen oder Aktivitäten von Gerinnungsfaktoren, (b) Mutanten von Gerinnungsfaktoren, (c) sehr geringe oder sehr hohe Konzentrationen oder Aktivitäten von Inhibitoren, (d) Mutanten von Inhibitoren oder (e) Antikörper gegen Elemente des Gerinnungssystems.

Im klinischen Alltag werden globale Screeningteste primär zur Diagnose hämorrhagischer oder thrombophiler Diathesen eingesetzt wie auch zur Überwachung von Therapien mit Medikamenten, die das Gerinnungssystem beeinflussen. Die Bestimmung der APTT zum Beispiel dient einerseits dem Screening auf Defekte des Teiles der Gerinnungskaskade, der über den sogenannten intrinsischen Weg gestartet wird und im gemeinsamen Weg aufgeht, und welcher aus den Gerinnungsfaktoren F VIII, F IX, F XI, F XII, Praekallikrein, HMW Kininogen, F V, F X, F II und Fibrinogen besteht. Ein APTT-Ergebnis oberhalb des Normalbereiches, d.h. eine verlängerte Gerinnungszeit kann auf einen Defekt eines oder mehrerer dieser Faktoren, zum Beispiel auf einen F VIII-Defekt, auch bekannt als Hämophilie A, hindeuten. Andererseits reagiert die APTT auch empfindlich auf die Anwesenheit von Antikoagulanzien, wie z.B. von Heparin, und wird daher auch zur Überwachung von Heparintherapien verwendet.

Die Bestimmung der PT (Prothrombinzeit, Quick-Test) dient dem Screening auf Defekte des Teiles der Gerinnungskaskade, der über den sogenannten extrinsischen Weg gestartet wird und im gemeinsamen Weg aufgeht, und welcher aus den Gerinnungsfaktoren F VII, F X, F II und Fibrinogen besteht. Weniger empfindlich erfasst der Test auch F V. Die PT-Bestimmung wird in erster Linie eingesetzt, um die Antikoagulanztherapie mit Cumarinen zu überwachen, um einen Vitamin K-Mangel zu erfassen oder zur allgemeinen Beurteilung des Gerinnungspotenzials, z.B. perioperativ oder bei Krankheitsverläufen.

Bei den chromogenen Testen wird die zu untersuchende Patientenprobe, die üblicherweise aus Plasma besteht, mit einem Gerinnungsaktivator und mit einem Substrat für einen Gerinnungsfaktor vermischt. Da es sich bei den meisten Blutgerinnungsfaktoren um Serin-Endopeptidasen handelt, also um Hydrolasen, die Peptidbindungen spalten können, werden vorwiegend Peptidsubstrate verwendet, die möglichst spezifisch von dem zu bestimmenden Blutgerinnungsfaktor gespalten werden und die eine nachweisbare Signalgruppe aufweisen. Bevorzugterweise werden abspaltbare chromogene oder fluorogene Signalgruppen verwendet, die optisch bestimmt werden.

Insbesondere mit Hilfe der chromogenen Teste können auch Antikoagulanzien, die die Aktivität von Blutgerinnungsfaktoren inhibieren, in Patientenproben bestimmt werden. Dazu wird die zu untersuchende Patientenprobe mit einem aktivierten Gerinnungsfaktor und mit einem Substrat für diesen Gerinnungsfaktor vermischt. Je mehr Antikoagulanz in der Probe enthalten ist, desto stärker wird der aktivierte Gerinnungsfaktor inhibiert und desto weniger Substrat wird gespalten.

Für die Beurteilung des Gerinnungsstatus eines Patienten bzw. bis zur Diagnose einer spezifischen Erkrankung, z.B. eines Faktormangels ist eine stufenweise Diagnostik notwendig, die üblicherweise mit einem Globaltest beginnt, gefolgt von zunehmend spezifischeren Testen.

Dies hat den Nachteil, dass die Diagnose einer Störung des Blutgerinnungssystems langwierig ist, dass viele verschiedene Reagenzien eingesetzt werden müssen, wodurch hohe Kosten entstehen und dass relativ viel Probenmaterial benötigt wird, was insbesondere bei Kleinkindern problematisch sein kann.

Es ist daher wünschenswert ein Verfahren bereit zu stellen, das die simultane Bestimmung mehrerer Parameter des Blutgerinnungsystems in einem einzigen Reaktionsansatz ermöglicht.

Es ist grundsätzlich bekannt, dass die Massenspektrometrie die simultane Bestimmung mehrerer Parameter in einem einzigen Testansatz ermöglicht (Multiplexierung). Im klinischen Routinelabor wird die Massenspektrometrie für das Drogenscreening, für die toxikologische Analytik oder die Steroidanalytik verwendet.

Bislang wurde für die Gerinnungsdiagnostik jedoch noch kein Parameter beschrieben, der sich für die massenspektrometrische Bestimmung der Aktivität des extrinsischen und/oder des intrinsischen Blutgerinnungssystems eines Individuums eignet, d.h. der in analoger Weise wie die PT oder die APTT mehrere enzymatische Reaktionen innerhalb bestimmter Bereiche des Blutgerinnungsystems erfasst.

Es wurde gefunden, dass die quantitative Bestimmung der Fibrinogenspaltprodukte Fibrinopeptid A und/oder Fibrinopeptid B in einer Probe eines Patienten, in der die Blutgerinnung in vitro aktiviert wurde, die Bestimmung der Aktivität des extrinsischen und/oder des intrinsischen Blutgerinnungssystems ermöglicht, denn die Menge von Fibrinopeptid A und auch die Menge von Fibrinopeptid B, die in dem Reaktionsansatz gebildet werden, verhalten sich umgekehrt proportional zur PT bzw. APTT des Patienten.

Die Bestimmung von in vivo gebildetem Fibrinopeptid A und B in einer Plasmaprobe eines Patienten ist bekannt. Erhöhte Fibrinopeptid A- oder Fibrinopeptid B-Konzentrationen zeigen eine verstärkte Thrombinaktivität an und indizieren Gerinnungsstörungen wie disseminierte intravasale Koagulopathien (DIC) und Thromboembolien (Cronlund, M. et al., Fibrinopeptide A in plasma of normal subjects and patients with disseminated intravascular coagulation and systemic lupus erythematosus. The Journal of Clinical Investigation 1976, 58: 142-151; Yudelman, I.M. et al., Plasma fibrinopeptide A levels in symptomatic venous thromboembolism. Blood 1978, 51(6): 1189-1195; Eckhardt, T. et al., Measurement of desarginine fibrinopeptide B in human blood. J. Clin. Invest. 1981, 67: 809-816). Normale in vivo-Fibrinopeptid A-Konzentrationen im Plasma liegen unter 2 ng/mL. Pathologische in vivo-Fibrinopeptid A-Konzentrationen liegen zwischen 3 und 20 ng/mL. Diese in vivo-Spiegel stören jedoch nicht die erfindungsgemäße Bestimmung der Aktivität des extrinsischen oder des intrinsischen Blutgerinnungssystems eines Individuums anhand der Menge der in vitro gebildeten Fibrinopeptid A-Menge, weil bei der in vitro-Aktivierung mittels geeigneter Aktivatoren des extrinsischen oder des intrinsischen Gerinnungsystems wesentlich höhere Fibrinopeptid A-Mengen freigesetzt werden.

Gegenstand der vorliegenden Erfindung ist daher ein Verfahren zur Bestimmung der Aktivität des extrinsischen oder des intrinsischen Blutgerinnungssystems eines Individuums umfassend die folgenden Schritte in der genannten Reihenfolge:
a) Vermischen einer Probe des Individuums und eines Aktivators des extrinsischen oder des intrinsischen Gerinnungsystems zu einem Reaktionsansatz,
b) Inkubation des Reaktionsansatzes und
c) Bestimmung der Menge von Fibrinopeptid A und/oder Fibrinopeptid B im Reaktionsansatz.

Die Bestimmung der Menge von Fibrinopeptid A und/oder Fibrinopeptid B im Reaktionsansatz kann beispielsweise mit Hilfe spezifischer, bevorzugterweise monoklonaler Antikörper erfolgen. Besonders bevorzugt erfolgt die quantitative Bestimmung massenspektrometrisch.

Unter dem Begriff "Fibrinopeptid A" (FPA) ist ein Oligopeptid mit der Aminosäuresequenz ADSGEGDFLAEGGGVR (SEQ ID NO: 1) zu verstehen, das unter der Einwirkung von Thrombin von humanem Fibrinogen abgespalten wird, beziehungsweise ein Fragment davon, welches mindestens sechs aufeinanderfolgende Aminosäurereste der Aminosäuresequenz aufweist. Das vollständige humane Fibrinopeptid A (SEQ ID NO: 1) besitzt einfach protoniert im ESI-Massenspektrum, ein Masse-zu-Ladung-Verhältnis (m/z) 1536,6929. Weitere Fragmente, vor allem das Des-Alanin FPA-Peptid mit der Aminosäuresequenz DSGEGDFLAEGGGVR (SEQ ID NO: 2) mit einem Masse-zu-Ladung-Verhältnis (m/z) 1465,6558 (einfach protoniert), können typischerweise auch im Blut oder Plasma detektiert werden. Sowohl das vollständige FPA-Peptid (SEQ ID NO: 1) als auch das Des-Alanin FPA-Peptid (SEQ ID NO: 2) können auch als Serin-phosphorylierte Formen ADS-pGEGDFLAEGGGVR mit einem berechneten Masse-zu-Ladung-Verhältnis von (m/z) 1616,659 (einfach protoniert) bzw. DS-pGEGDFLAEGGGVR mit einem Masse-zu-Ladung-Verhältnis (m/z) 1545,622 (einfach protoniert) vorliegen. Die Berechnung der monoisotopischen Masse von FPA und allen folgenden Peptiden in der Elektrospray-Serie erfolgte mittels Peptide Mass Calculator v3.2 des Rega Institute for Medical Research - Leuven (Belgien), oder mittels Datenanalysesoftware Xcalibur SW-Version 2.10 von Thermo Fisher Scientific.

Unter dem Begriff "Fibrinopeptid B" (FPB) ist ein Oligopeptid mit der Aminosäuresequenz QGVNDNEEGFFSAR (SEQ ID NO: 3) zu verstehen, das unter der Einwirkung von Thrombin von humanem Fibrinogen abgespalten wird, beziehungsweise ein Fragment davon, welches mindestens sechs aufeinanderfolgende Aminosäurereste der Aminosäuresequenz aufweist. Das vollständige humane Fibrinopeptid B (SEQ ID NO: 3) besitzt einfach protoniert ein Masse-zu-Ladung-Verhältnis (m/z) 1569,6933. Weitere im Blut oder Plasma vorkommende FPB-Fragmente sind EGVNDNEEGFFSAR (SEQ ID NO: 4) mit einem Masse-zu-Ladung-Verhältnis (m/z) 1570,6773, ZGVNDNEEGFFSAR mit N-terminaler Pyroglutaminsäure (Abkürzung "Z") und einem Masse-zu-Ladung-Verhältnis (m/z) 1552,6663 oder der des-Arg-Fibrinopeptid B-Metabolit mit der Aminosäuresequenz QGVNDNEEGFFSA (SEQ ID NO: 5) und einem Masse-zu-Ladung-Verhältnis (m/z) 1413,5922 oder ZGVNDNEEGFFSA mit einem Masse-zu-Ladung-Verhältnis (m/z) 1395,5578, jeweils einfach protoniert.

Unter einem "Aktivator des extrinsischen Gerinnungsystems" ist eine Substanz oder ein Substanzgemisch zu verstehen, die/das durch Zugabe zu einer humanen Plasmaprobe den extrinsischen Weg des Blutgerinnungssystems aktiviert. Bevorzugte Aktivatoren sind Gemische aus Gewebefaktor (tissue factor), Phospholipiden (Thromboplastine) und Calciumchlorid. Der Gewebefaktor kann aus Hirn, Lunge oder Plazentagewebe eines Säugetiers stammen (z.B. Mensch oder Kaninchen), oder er kann alternativ rekombinant oder synthetisch hergestellt sein. Alternativ können auch gerinnungsaktivierende Schlangengifte oder eine aus Schlangengift isolierte gerinnungsaktivierende Protease als Aktivator eingesetzt werden.

Unter einem "Aktivator des intrinsischen Gerinnungsystems" ist eine Substanz oder ein Substanzgemisch zu verstehen, die/das durch Zugabe zu einer humanen Plasmaprobe den intrinsischen Weg des Blutgerinnungssystems aktiviert. Bevorzugte Aktivatoren sind Phospholipide und negativ geladene Oberflächen wie Glas, Silica, Kaolin, Ellagsäure, Celit und Plättchenfaktor 3. Durch Zugabe von Calciumchlorid wird der Gerinnungsvorgang ausgelöst.

Für die massenspektrometrische Bestimmung/Messung des Fibrinopeptids A und/oder B können alle Arten von Massenspektrometern verwendet werden, die geeignet sind, Substanzen in die Gasphase zu überführen, zu ionisieren und deren Masse zu messen. Anhand des Masse-zu-Ladung-Verhältnisses (m/z) kann eine Substanz identifiziert und quantifiziert werden. Insbesondere geeignet sind Quadrupol-Massenspektrometer, Flugzeitmassenspektrometer (TOFMS) und Ionenfallen-Massenspektrometer (Quadrupol-Ionenfalle, Linear Trap, Fourier-Transformations-Ionenzyklotronresonanz (FT-ICR), Orbitrap mit Elektrosprüh-Ionenquellen (ESI, DESI), oder mit Ionisierung durch matrixunterstützte Laserdesorption (MALDI). Die Ionisierung der Probe kann aber auch mit Atmospheric Pressure Photo Ionization (APCI), mit Elektronenstoßionisation (EI), mit chemischer Ionisation (CI), Photoionisation (PI , APPI), Feldionisation (FI), Fast Atom Bombardment (FAB), Secondary Ion MS (SIMS) und Plasma Desorption erfolgen. Die Kombination mit einem Flüssigchromatographieverfahren (LC) kann zum Zwecke der Sensitivitätserhöhung des Verfahrens vor der eigentlichen massenspektrometrischen Analyse eingesetzt werden, ist aber nicht darauf beschränkt.

Unter dem Begriff "Probe eines Individuums" ist eine Plasma- oder Vollblut-Probe zu verstehen, die bei Entnahme mit einem Antikoagulanz wie Citrat oder EDTA zur Vermeidung einer spontanen, unkontrollierten Gerinnung vermischt wurde. Die Probe kann vorbehandelt sein, also vor der Durchführung des erfindungsgemäßen Verfahrens einem Verfahren unterworfen worden sein, das der Entfernung abundanter hochmolekularer Substanzen, insbesondere von Proteinen dient, wie beispielsweise einer Affinitätschromatographie oder einer Proteinfällung durch Zugabe z.B. von Methanol, Aceton, Acetonitril, einer Säure oder Lauge mit anschließender Abtrennung des unerwünschten Proteinanteils, z.B. durch Zentrifugation oder Filtration. Weitere Verfahren zur Vorbehandlung der Proben umfassen die Anreicherung der nachzuweisenden Probenbestandteile durch reversible Extraktion aus der flüssigen Phase mittels bindungsaktiver Oberflächen, wie z.B. Reversed Phase-, Anionen- oder Kationenaustauscher-Oberflächen, oder einer mit Antikörpern, Aptameren oder Gerinnungsinhibitoren beschichteten Oberfläche.

Es wurde jedoch gefunden, dass insbesondere bei der Anwendung massenspektrometrischer Bestimmungsmethoden eine besondere Probenvorbehandlung nicht notwendigerweise erfolgen muss. Die massenspektrometrische Bestimmung von Fibrinopeptid A und/oder B ist überraschenderweise auch in Plasma- oder Vollblutproben möglich bzw. in Reaktionsansätzen, wie sie für das erfindungsgemäße Verfahren aus der Probe und dem Aktivator gemischt werden, die weder einem Verfahren zur Entfernung von Proteinen noch einem tryptischen Verdau unterworfen wurden.

Bei der Verwendung von Plasma- oder Vollblutproben, die weder einem Verfahren zur Entfernung von Proteinen noch einem tryptischen Verdau unterworfen wurden, als Probenmaterial wird die Probe vorzugsweise ferner mit einem Fibrinpolymerisationsinhibitor vermischt. Dies hat den Vorteil, dass kein störendes Fibringerinnsel im Reaktionsansatz entsteht.

Unter dem Begriff "Fibrinpolymerisationsinhibitor" ist eine Substanz, vorzugsweise ein Oligopeptid zu verstehen, die/das die Aneinanderlagerung (Polymerisation) der Fibrinmonomere, die unter der Einwirkung von Thrombin entstehen, inhibiert und damit die Gerinnselbildung verhindert (siehe z.B. EP-A2-0456152 A2). Besonders bevorzugte Fibrinpolymerisationsinhibitoren sind Oligopeptide der allgemeinen Formel GPRP-X-NH₂, wobei G für die Aminosäure Glycin, P für die Aminosäure L-Prolin, R für die Aminosäure L-Arginin und X für Alanin oder Glycin steht.

Nach dem Vermischen der Probe des Individuums und eines Aktivators des extrinsischen oder des intrinsischen Gerinnungsystems und gegebenenfalls eines Fibrinpolymerisationsinhibitors zu einem Reaktionsansatz wird der Reaktionsansatz für eine gewisse Zeit, vorzugsweise für eine Zeit von 1 bis 120 Sekunden, bei einer Temperatur zwischen etwa 15-40 °C, vorzugsweise bei 37 °C inkubiert.

Bevor die Menge von Fibrinopeptid A und/oder Fibrinopeptid B im Reaktionsansatz gemessen wird, wird die Inkubation des Reaktionsansatzes bevorzugterweise beendet, d.h. die Reaktionen im Reaktionsansatz werden abgestoppt. Die Inkubation des Reaktionsansatzes kann durch Zugabe von Methanol, Aceton, Acetonitril, einer Säure oder einer Lauge oder durch Erhitzen des Reaktionsansatzes auf eine Temperatur zwischen 60 und 100 °C beendet werden. Alternativ kann die Inkubation des Reaktionsansatzes durch reversible Extraktion aus der flüssigen Phase an bindungsaktive Oberflächen, z.B. an einer Reversed Phase-, Anionen- oder Kationenaustauscher-Oberfläche oder an einer mit Antikörpern beschichteten Oberfläche abgestoppt werden. Ferner kann die Inkubation des Reaktionsansatzes auch durch Zugabe eines Thrombininhibitors, wie z.B. von Hirudin, Aprotinin oder Rivaroxaban, oder durch Zugabe eines Inhibitors der Fibrinolyse, z.B. eines Plasmininhibitors, wie z.B. alpha-2-anti-Plasmin, Aprotinin und Tranexamsäure, oder durch Einfrieren, durch Filtration oder durch Trocknung des Reaktionsansatzes abgestoppt werden.

Vorzugsweise wird dem Reaktionsansatz, bevor die Menge von Fibrinopeptid A und/oder Fibrinopeptid B im Reaktionsansatz gemessen wird, zusätzlich mindestens 1 Volumenteil Wasser, bevorzugt A. dest. zugegeben. Dies hat den Vorteil, dass eine bessere Ionisierung der Probenbestandteile und eine niedrigere Gesamtproteinkonzentration im Reaktionsansatz erreicht wird, wodurch die massenspektrometrische Bestimmung verbessert wird.

In einer Ausführungsform des erfindungsgemäßen Verfahrens wird die Inkubation des Reaktionsansatzes beendet, indem das Erhitzen des Reaktionsansatzes auf eine Temperatur zwischen 60 und 100 °C durch Zugabe von mindestens 1 Volumenteil Wasser erzielt wird, welches eine Temperatur zwischen 60 und 100 °C aufweist.

Weiterhin bevorzugte Ausführungsformen des erfindungsgemäßen Verfahrens betreffen die massenspektrometrische Bestimmung der Aktivität des extrinsischen oder des intrinsischen Blutgerinnungssystems eines Individuums durch die Bestimmung von Fibrinopeptid A und/oder B im Reaktionsansatz und zusätzlich der Menge eines oder mehrerer therapeutischer Antikoagulanzien und/oder der Menge mindestens eines weiteren Aktivierungspeptids des Blutgerinnungssystems und/oder der Menge mindestens eines weiteren Fibrinogenspaltproduktes und/oder der Menge mindestens eines Markers der Plättchenaktivierung und/oder der Menge mindestens eines Fibrinspaltproduktes im selben Reaktionsansatz.

Die zusätzliche Bestimmung der Menge eines oder mehrerer therapeutischer Antikoagulanzien umfasst insbesondere die Bestimmung von therapeutischen Antikoagulanzien aus der Gruppe Hirudin, Dabigatran, Melagatran, Argatroban, Ximelagatran, Bivalirudin, Lepirudin, MCC-977, SSR-182289, TGN-255, TGN-167, ARC-183 Odiparcil, Rivaroxaban, Apixaban, Edoxaban, Otamixaban,LY 517717, YM 153, DU-176b, DX-9065a und KFA-1982, Heparin, Warfarin und Phenprocoumon.

Die zusätzliche Bestimmung der Menge mindestens eines weiteren Aktivierungspeptids des Blutgerinnungssystems umfasst insbesondere die Bestimmung von Aktivierungspeptiden aus der Gruppe Faktor XIII-Aktivierungspeptid, Faktor X-Aktivierungspeptid, Faktor IX-Aktivierungspeptid und Protein C-Aktivierungspeptid.

Unter dem Begriff "Faktor XIII-Aktivierungspeptid" (F XIII-AP) ist ein Oligopeptid mit der wildtypischen Aminosäuresequenz SETSRTAFGGRRAVPPNNSNAAEDDLPTVELQGVVPR (SEQ ID NO: 6) mit einem Masse-zu-Ladung-Verhältnis (m/z) 3949,9755 (einfach protoniert) oder der mutierten V34L-Variante mit der Aminosäuresequenz SETSRTAFGGRRAVPPNNSNAAEDDLPTVELQGLVPR (SEQ ID NO: 7) und einem Masse-zu-Ladung-Verhältnis (m/z) 3963,9912 (einfach protoniert) zu verstehen, die jeweils unter der Einwirkung von Thrombin von humanem Faktor XIII abgespalten werden. Die Bestimmung der Menge des Faktor XIII-Aktivierungspeptids im Reaktionsansatz ermöglicht die Bestimmung der in der Probe enthaltenen Faktor XIII-Menge/-Aktivität. Die physiologische Funktion des Faktors XIII besteht in der Stabilisierung des Fibrins durch die kovalente Quervernetzung der beim Gerinnungsvorgang entstehenden Fibrinmonomere. Da die Faktor XIII-Aktivität erst am Ende der Gerinnungskaskade, mit Einsetzen der Fibrinbildung zum Tragen kommt, ist ein Faktor XIII-Mangel mit üblichen Gerinnungstesten, die auf der Bestimmung der Fibrinbildungszeit beruhen, nicht erfassbar.

Die zusätzliche Bestimmung der Menge mindestens eines weiteren Fibrinogenspaltproduktes umfasst insbesondere die Bestimmung des Fibrinogenspaltproduktes Bß₁₋₄₂-Peptid. Unter dem Begriff "Bß₁₋₄₂-Peptid" ist ein Oligopeptid mit der Aminosäuresequenz QGVNDNEEGFFSARGHRPLDKKREEAPSLRPAPPPISGGGYR (SEQ ID NO: 8) zu verstehen, das unter der Einwirkung von Thrombin von humanem Fibrinogen abgespalten wird. Das berechnete Masse-zu-Ladung-Verhältnis der positiven ESI-Serie für das Bß₁₋₄₂-Peptid ist wie folgt:

| Ladung | m/z |
|---|---|
| +1 | 4589,3149 |
| +2 | 2295,1613 |
| +3 | 1530,4435 |
| +4 | 1148,0845 |
| +5 | 918,6692 |
| +6 | 765,7256 |
| +7 | 656,4802 |
| +8 | 574,5462. |

Die zusätzliche Bestimmung der Menge mindestens eines Markers der Plättchenaktivierung umfasst insbesondere die Bestimmung von 5-Hydroxytryptamin (Serotonin) mit einem Masse-zu-Ladung Verhältnis (m/z) 177,22, ß-Thromboglobulin oder Plättchenfaktor 4.

Die zusätzliche Bestimmung der Aktivität des Fibrinolysesystems des Individuums, indem in dem Reaktionsansatz zusätzlich die Menge mindestens eines Fibrinspaltproduktes bestimmt wird, umfasst insbesondere die Bestimmung des Fibrinspaltproduktes Bβ₁₅₋₄₂-Peptid. Unter dem Begriff "Bß₁₅₋₄₂-Peptid" ist ein Oligopeptid mit der Aminosäuresequenz GHRPLDKKREEAPSLRPAPPPISGGGYR (SEQ ID NO: 9) zu verstehen, das unter der Einwirkung von Thrombin von humanem Fibrin abgespalten wird. Das Bß₁₅₋₄₂-Peptid hat ein Masse-zu-Ladung-Verhältnis (m/z) 3038,6399 (einfach protoniert).

Die Vorteile des erfindungsgemäßen Verfahrens zur globalen Bestimmung der Aktivität des extrinsischen oder des intrinsischen Blutgerinnungssystems eines Individuums bestehen darin, dass im Falle der massenspektrometrischen Bestimmung eine hohe Sensitivität und Selektivität erzielt wird und dass in einem einzigen Reaktionsansatz zusätzlich zu der globalen Aktivität des Blutgerinnungssystems zahlreiche weitere Gerinnungs- und/oder Fibrinolyseparameter bestimmt werden können. Eine derartige multiplexe Messung globaler und spezifischer Gerinnungs- und Fibrinolyseparameter ist im Stand der Technik nicht bekannt.

### FIGURENBESCHREIBUNG

### Figur 1

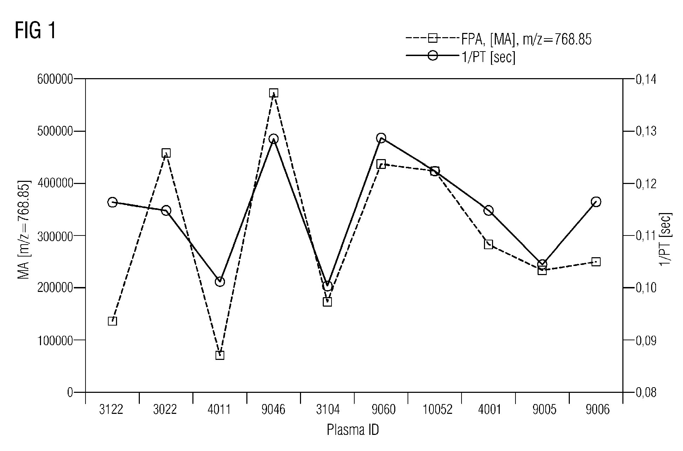

Figur 1 zeigt die Gegenüberstellung der konventionell bestimmten Prothrombinzeit (1/PT in Sekunden) und der massenspektrometrisch bestimmten Fibrinopeptid A-Menge (FPA) (integrierte Peakfläche (MA) von m/z 768,85), in mit Innovin®-Reagenz aktivierten Citratplasmaproben von 10 verschiedenen Spendern (Inkubationszeit 30 Sekunden).

### Figur 2

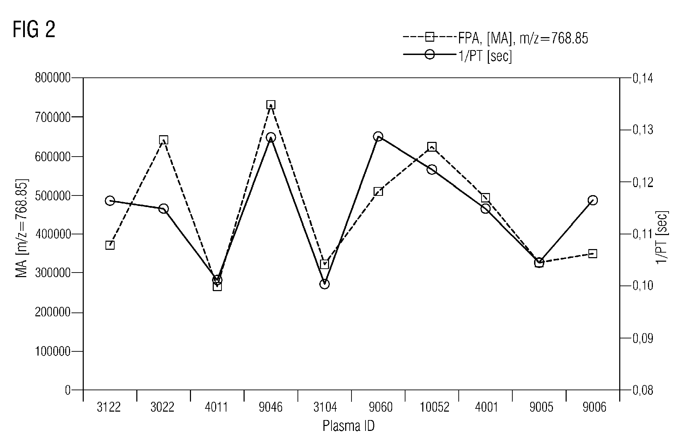

Figur 2 zeigt die Gegenüberstellung der konventionell bestimmten Prothrombinzeit (1/PT in Sekunden) und der massenspektrometrisch bestimmten Fibrinopeptid A-Menge (FPA) (integrierte Peakfläche (MA) von m/z 768,85), in mit Innovin®-Reagenz aktivierten Citratplasmaproben von 10 verschiedenen Spendern (Inkubationszeit 60 Sekunden).

### Figur 3

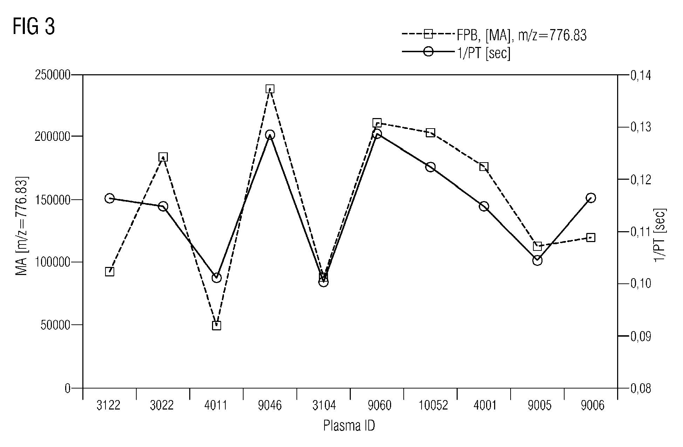

Figur 3 zeigt die Gegenüberstellung der konventionell bestimmten Prothrombinzeit (1/PT in Sekunden) und der massenspektrometrisch bestimmten Fibrinopeptid B-Menge (FPB) (integrierte Peakfläche (MA) von m/z 776,83), in mit Innovin®-Reagenz aktivierten Citratplasmaproben von 10 verschiedenen Spendern (Inkubationszeit 60 Sekunden).

### BEISPIELE

### BEISPIEL 1: Massenspektrometrische Quantifizierung von Fibrinopeptid A und Fibrinopeptid B in aktivierten Plasmaproben zur Bestimmung der Aktivität des extrinsischen Blutgerinnungssystems

In einem Reaktionsgefäß wurden jeweils 15 µL humanes Citratplasma von 10 Spendern 1:10 in Wasser enthaltend H-Gly-Pro-Arg-Pro-NH₂ als Fibrinpolymerisationsinhibitor verdünnt und mit 30 µL Innovin®-Reagenz, einem Thromboplastinreagenz enthaltend humanen, rekombinanten Gewebefaktor, synthetische Phospholipide, eine Heparin-neutralisierende Verbindung, Puffer und Rinderserumalbumin (Siemens Healthcare Diagnostics Products GmbH, Marburg, Deutschland), zu einem Reaktionsansatz vermischt. Als Negativkontrolle wurde anstelle des Thromboplastinreagenzes Wasser Zur Probenaktivierung verwendet.

Nach 0, 15, 30 und 60 Sekunden Inkubation bei +37 °C wurden je 5 µL des Reaktionsansatzes entnommen und mit 20 µL Wasser, temperiert auf 99 °C, vermischt, um die Reaktion abzustoppen. Alternativ wurden 5 µL der Reaktionsansätze mit 20 µL Methanol vermischt, um die Reaktion abzustoppen und anschließend bei 6400 UPM für 30 Sekunden zentrifugiert.

Jeweils 10 µL der so behandelten Reaktionsansätze wurden mittels Autosampler in die LC ESI-MS injiziert und unter folgenden Bedingungen gemessen:

### Flüssigkeitschromatographische Bedingungen

Die Analyse der Proben erfolgte auf einem Agilent 1100 HPLC System und einem gekoppelten LTQ-FT Massenspektrometer von Thermofisher Scientific. Die chromatographische Auftrennung der Proben erfolgte mittels Nucleodur® EC50/3-125-2-C18ec 3 µm Reverse Phase Säule von Macherey & Nagel, bei einer Säulentemperatur von 40 °C und einer Flussrate von 0,2 mL/min. Die mobile Phase bestand aus Laufmittel A: Wasser mit 0,1 % Ameisensäure und Laufmittel B: Acetonitril mit 0,1 % Ameisensäure. Der Gradient war wie folgt: 0 min: 5% B; 4 min: 95% B; 8 min: 95% B; 8,2 min: initiale Bedingungen bis 15 min. Die UV-Detektion erfolgte bei 215 nm. Alternativ wurde eine Phenomenex Kinetex 2,6 µm - C18 - 100 A, 150*3 mm LC-Säule verwendet mit identischen Bedingungen für Laufmittel, Temperatur, Flussrate und UV-Detektion. Der Gradient war für diese Probenanalyse wie folgt: 0 min: 5% B; 15 min: 95 % B; 16 min: 95% B; ab 17 min. initiale Bedingungen bis 25 min.

### Massenspektrometrische Bedingungen:

Für die Detektion von Fibrinopeptid A (FPA) und Fibrinopeptid B (FPB) in den mittels Flüssigkeitschromatographie getrennten Reaktionsansätzen wurde ein LTQ-FT-Massenspektrometer von Thermo Fisher Scientific verwendet. Das LTQ-FT ist ein Hybridmassenspektrometer, welches die lineare Ionenfalle mit der hochauflösenden Fourier-Transformation-Ionen-Zyklotron-Technik kombiniert. Die Ionisierung der Proben erfolgte mittels Elektrospray-Ionisation (ESI) im positiven Ionisationsmodus. Die Proben wurden auf FPA unter den folgenden für die Peptide mit den Sequenzen SEQ ID Nos: 1-2 und auf FPB unter den für die Peptide mit den Sequenzen SEQ ID Nos: 3-5 berechneten Masse-zu-Ladung-Verhältnissen der Elektrospray-Ionisations-Serie im Massenbereich (m/z) 200 bis 2000, mit einer Auflösung R = 100.000 gescannt. Unter der hier beschriebenen massenspektrometrischen Methode zeigte FPA einen dominierenden Ionenpeak bei m/z = 768,8504 und FPB bei m/z 785,8425 und 776,8368 (zweifach protoniert).

Die Berechnung der monoisotopischen Masse der Peptide der Elektrospray-Serie (positive Mode) erfolgte mittels Peptide Mass Calculator v3.2 des Rega Institute for Medical Research - Leuven (Belgien), oder mittels Datenanalysesoftware Xcalibur SW-Version 2.10 von Thermo Fisher Scientific. Das berechnete Masse-zu-Ladung-Verhältnis der positiven Elektospray-Ionisierungs-Serie für FPA ist wie folgt:
ESI-Serie des FPA, SEQ ID NO: **1:** (ADSGEGDFLAEGGGVR)

| Ladung | m/z |
|---|---|
| +1 | 1536,6929 |
| +2 | 768,8504 |
| +3 | 512,9028 |
| +4 | 384,9291 |

ESI-Serie des FPA, SEQ ID NO: 2: (DSGEGDFLAEGGGVR)

| Ladung | m/z |
|---|---|
| +1 | 1465,6558 |
| +2 | 733,3318 |
| +3 | 489,2238 |
| +4 | 367,1698 |

ESI-Serie des Serin-phosphorylierten FPA, (ADS-pGEGDFLAEGGGVR)

| Ladung | m/z |
|---|---|
| +1 | 1616,659 |
| +2 | 808,834 |
| +3 | 539,558 |

ESI-Serie des Serin-phosphorylierten FPA, (DS-pGEGDFLAEGGGVR)

| Ladung | m/z |
|---|---|
| +1 | 1545,622 |
| +2 | 773,315 |
| +3 | 515,879 |

Das berechnete Masse-zu-Ladung-Verhältnis der positiven ESI-Serie für humanes FPB und seine Varianten oder Metabolite ist wie folgt:
ESI-Serie des FPB, SEQ ID NO: 3 (QGVNDNEEGFFSAR)

| Ladung | m/z |
|---|---|
| +1 | 1569,6933 |
| +2 | 785,3505 |
| +3 | 523,9029 |

ESI-Serie des FPB, SEQ ID NO: 4 (EGVNDNEEGFFSAR)

| Ladung | m/z |
|---|---|
| +1 | 1570,6773 |
| +2 | 785,8425 |
| +3 | 524,2309 |

ESI-Serie des Pyroglutamat-FPB, (ZGVNDNEEGFFSAR)

| Ladung | m/z |
|---|---|
| +1 | 1552,6663 |
| +2 | 776,8368 |
| +3 | 517,8915 |

ESI-Serie des FPB, SEQ ID NO: 5 (QGVNDNEEGFFSA)

| Ladung | m/z |
|---|---|
| +1 | 1413,5922 |
| +2 | 707,3000 |
| +3 | 471,8692 |

ESI-Serie des Pyroglutamat-FPB, (ZGVNDNEEGFFSA)

| Ladung | m/z |
|---|---|
| +1 | 1395,5578 |
| +2 | 698,2828 |
| +3 | 465,8578 |

### Allgemeine MS-Parameter

Im Folgenden sind die ESI- und MS-Parameter zusammengestellt, welche sowohl für die Identifizierung als auch für die Quantifizierung verwendet wurden:

### Ionenquelle ESI:

Positive Polarity
Sheath Gas Flow () 30.00
Aux Gas Flow () 20
Capillary Temperature (°C) 320.00
Source Voltage (kV) 3.80
Source Current (µA) 100.00
Source Fragmentation -10 V bzw. 10 V
Tube Lens (V) 160.00
Skimmer Offset (V) 0.00
Multiple RF Amplifier (Vp-p) 400.00
Multipole 00 Offset (V) -3.32
Capillary Voltage (V) 4.00

### LTQ Ion Trap/FTMS (n) :

| | |
|---|---|
| Mass Range (m/z): | 200-2000 |
| Ion Trap Injection Waveform: | off |
| Ion Trap Zoom AGC Target: | 2000.00 |
| Ion Trap Full AGC Target: | 30000.00 |
| Ion Trap SIM AGC Target: | 10000.00 |
| Ion Trap MSn AGC Target: | 10000.00 |
| FTMS ECD AGC Target: | 400000.00 |
| FTMS Injection Waveform: | off |
| FTMS Full Scan AGC Target (m(max. Ionenzahl): | 1000000.00 |
| FTMS Narrow SIM AGC Target: | 100000.00 |
| FTMS Wide SIM AGC Target: | 500000.00 |
| FTMS MSn AGC Target: | 1000000.00 |
| FTMS ECD AGC Target: | 400000.00 |
| Data Type: centroid | |
| Mass Calibration extern | |
| | |
| Lens 0 Voltage (V): | -6.50 |
| Multipole 0 Offset (V) | -7.50 |
| Lens 1 Voltage (V): | -18.50 |
| Gate Lens Offset (V) | -64.00 |
| Multipole 1 Offset (V) | -6.00 |
| Front Lens (V): | -6.75 |
| | |
| Ion Trap Zoom Micro Scans: | 1 |
| Ion Trap Zoom Max Ion Time(ms) : | 50.00 |
| Ion Trap Full Micro Scans: | 1 |
| Ion Trap Full Max Ion Time (ms): | 100.00 |
| Ion Trap Narrow SIM Micro Scans: | 1 |
| Ion Trap Narrow SIM Max Ion Time(ms): | 50.00 |
| Ion Trap Wide SIM Micro Scans: | 1 |
| Ion Trap Wide SIM Max Ion Time (ms): | 500.00 |
| Ion Trap MSn Micro Scans: | 1 |
| Ion Trap MSn Max Ion Time (ms): | 2500.00 |
| FTMS Full Micro Scans: | 1 |
| FTMS Full Max Ion Time (ms): | 2500.00 |
| FTMS SIM Micro Scans: | 1 |
| FTMS SIM Max Ion Time (ms): | 1000.00 |
| FTMS MSn Micro Scans: | 1 |
| FTMS MSn Max Ion Time (ms): | 500.00 |
| FTMS ECD Micro Scans: | 1 |
| FTMS ECD Max Ion Time (ms): | 1000.00 |

Für die qualitative und quantitative Auswertung der Massenspektren diente die Steuerungs- und Datenanalysesoftware Xcalibur SW-Version 2.10 von Thermo Fisher Scientific.

### Quantifizierung von FPA und FPB und Ermittlung der Sensitivität der Methode

Zur quantitativen Konzentrationsbestimmung von FPA wurde eine Kalibrationskurve von humanem Fibrinopeptid A erstellt. Dazu wurde humanes Fibrinopeptid A (≥97% (HPLC), chemische Formel: C₆₃H₉₇N₁₉O₂₆, Molare Masse 1536.56, Sigma-Aldrich Chemie GmbH, Steinheim, Deutschland) in Wasser oder in einem Citratplasmapool in den Konzentrationen 0, 500, 1.000, 5.000, 10.000, 30.000, 50.000 und 100.000 µg/L gelöst, anschließend 1:100 in Wasser verdünnt und durch Injektion von 10 µL unter den oben beschriebenen LC ESI-MS-Bedingungen gemessen. Analog erfolgte die Präparation und Analyse der FPB Kalibrationskurve mit humanem Fibrinopeptid B (≥97% (HPLC), chemische Formel: C₆₆H₉₃N₁₉O₂₅, Molare Masse 1552.56, Sigma-Aldrich Chemie GmbH).

In Tabelle 1 sind die Messwerte für FPA in Wasser und in einem Citratplasmapool gezeigt. Für die Erstellung einer Kalibrationsgeraden wurde die FPA-Kalibrationskurve in Citratplasma verwendet. Es ist die integrierte Peakfläche (MA) von m/z 768,85 angegeben.

**Tabelle 1: Kalibrationsmesswerte für FPA**

| **FPA (µg/L)** | **FPA in A.dest. (MA)** | **FPA in Plasma (MA)** |
|---|---|---|
| 100 | 1145970 | 1392478 |
| 50 | 605102 | 755281 |
| 30 | 334015 | 432187 |
| 10 | 118123 | 144671 |
| 5 | 61029 | 76528 |
| 0 | 1105 | 6902 |

Zur Ermittlung der Fibrinopeptid-Konzentration (FPA, FPB) in den mit Innovin®-Reagenz aktivierten Reaktionsansätzen wurden die gemessenen, integrierten Peakflächen der Proben auf die erstellte Kalibrationsgerade abgetragen. Die Ermittlung der Nachweisgrenze der Methode erfolgte anhand der Kalibrationskurve. Wie in Tabelle 1 zu sehen liegt die Nachweisgrenze in Citratplasma unter 5 µg/L FPA.

Zur Bestimmung der Prothrombinzeit (PT) wurden Citratplasmaproben von 10 Spendern mit Innovin®-Reagenz vermischt, und es wurde die Gerinnungszeit in einem BCS® XP Gerät (Siemens Healthcare Diagnostics Products GmbH Marburg, Deutschland) optisch (photometrisch) bestimmt. Parallel hierzu wurden FPA und FPB wie in Beispiel 1 beschrieben nach Aktivierung derselben Citratplasmaproben massenspektrometrisch bestimmt.

In Tabelle 2 sind für jede mit Innovin®-Reagenz aktivierte und für 60 Sekunden inkubierte Citratplasmaprobe (Proben ID) die massenspektrometrisch bestimmten Fibrinopeptid A-Messwerte (integrierte Peakfläche(MA) von m/z = 768,85) und die über eine FPA-Kalibrationskurve berechneten FPA-Konzentrationen in µg/L, die massenspektrometrisch bestimmten Fibrinopeptid B-Messwerte (integrierte Peakfläche von m/z = 776,83 (MA)) sowie die optisch bestimmte Prothrombinzeit (PT in Sekunden) gegenüber gestellt.

**Tabelle 2: Vergleich der FPA- und FPB-Messwerte mit der Prothrombinzeit (PT)**

| **Proben-ID** | **FPA [MA]** | **FPA [µg/L]** | **FPB [MA]** | **PT [Sek]** |
|---|---|---|---|---|
| 3122 | 370945 | 25,74 | 92271 | 8.6 |
| 3022 | 639264 | 42,82 | 182920 | 8.7 |
| 4011 | 266134 | 18,45 | 49971 | 9.9 |
| 9046 | 727682 | 48,29 | 235957 | 7.8 |
| 3104 | 320140 | 22,21 | 87150 | 10.0 |
| 9060 | 507232 | 34,65 | 179997 | 7.8 |
| 10052 | 622586 | 41,79 | 211363 | 8.2 |
| 4001 | 488469 | 33,48 | 175850 | 8.7 |
| 9005 | 325474 | 22,58 | 112920 | 9.6 |
| 9006 | 346520 | 24,04 | 114393 | 8.6 |

Weitere Ergebnisse sind in den Figuren 1 bis 3 dargestellt, in welchen für jede Probe die konventionell bestimmte PT (1/PT) der FPA- bzw. der FBP-Menge gegenüber gestellt ist. Aus den Figuren 1-3 wird deutlich, dass sich die Menge von Fibrinopeptid A und auch die Menge von Fibrinopeptid B, die in dem Reaktionsansatz in vitro gebildet werden, umgekehrt proportional zur PT der Probe verhalten.

### BEISPIEL 2: Massenspektrometrische Quantifizierung von Fibrinopeptid A und Fibrinopeptid B in aktivierten Plasmaproben zur Bestimmung der Aktivität des intrinsischen Blutgerinnungssystems

Zur Bestimmung der APTT wurden Citratplasmaproben von 10 Spendern mit Pathromtin® SL Reagenz enthaltend einen Oberflächenaktivator und Phospholipide (Siemens Healthcare Diagnostics Products GmbH, Marburg, Deutschland) und CaCl₂-Lösung vermischt, und es wurde die Gerinnungszeit in einem BCS® XP Gerät (Siemens) photometrisch bestimmt.

Parallel hierzu wurden FPA und FPB wie folgt beschrieben nach Aktivierung derselben Citratplasmaproben mit Pathromtin® SL Reagenz massenspektrometrisch bestimmt. In einem Reaktionsgefäß wurden 30 µL Citratplasma, 30 µL Pathromtin® SL Reagenz oder als negative Kontrolle Wasser als Initiatorreagenz zugesetzt. Zusätzlich wurden 30 µL 25 mM CaCl₂-Lösung mit Zusatz von 6 mg/mL Fibrinaggregationshemmer H-Gly-Pro-Arg-Pro-NH₂ zugegeben und gemischt. Nach 0, 15, 30, 60 und 120 Sekunden Inkubation bei +37 °C wurden je 10 µL des Reaktionsansatzes mit 500 µL Wasser temperiert auf 99 °C gestoppt. Alternativ wurden die Proben mit 500 µL Methanol gestoppt und bei 13000 UPM für 60 Sekunden zentrifugiert. 10 µL der gestoppten Probe wurde mittels Autosampler in die LC ESI-MS injiziert und unter den in Beispiel 1 aufgeführten flüssigkeitschromatographischen und massenspektrometrischen Bedingungen quantifiziert.

In Tabelle 3 sind für jede mit Pathromtin® SL Reagenz aktivierte und für 60 Sekunden inkubierte Citratplasmaprobe (Proben ID) die massenspektrometrisch bestimmten Fibrinopeptid A-Messwerte (integrierte Peakfläche (MA) von m/z 768,85 und die über eine FPA-Kalibrationskurve berechneten FPA-Konzentrationen in µg/L der optisch bestimmten aktivierten partielle Thromboplastinzeit (APTT in Sekunden) gegenüber gestellt.

**Tabelle 3: Vergleich der FPA-Messwerte mit der Gerinnungszeit der APTT-Methode**

| **Proben-ID** | **FPA [MA]** | **FPA [µg/L]** | **APTT [Sek]** |
|---|---|---|---|
| 3122 | 236377 | 16,38 | 27,1 |
| 3022 | 265024 | 18,37 | 26,2 |
| 4011 | 201843 | 13,98 | 29,8 |
| 9046 | 227530 | 15,76 | 29,9 |
| 3104 | 117699 | 8,02 | 36,8 |
| 9060 | 249071 | 17,26 | 25,4 |
| 10052 | 228892 | 15,86 | 31,6 |
| 4001 | 179584 | 12,43 | 34,7 |
| 9005 | 119660 | 8,16 | 35,5 |
| 9006 | 197397 | 13,67 | 29,3 |

Aus Tabelle 3 wird deutlich, dass sich die Menge von Fibrinopeptid A, das in dem Reaktionsansatz in vitro gebildet wird, umgekehrt proportional zur APTT der Probe verhält.

### BEISPIEL 3: Massenspektrometrische Quantifizierung des Fibrinspaltproduktes Bβ₁₅₋₄₂ Peptid in Innovin®-aktivierten Plasmaproben nach Plasminzugabe

In einem Reaktionsgefäß wurden 50 µL Citratplasma 50 µL Innovin®-Reagenz bzw. als negative Kontrolle Wasser als Initiatorreagenz zugesetzt. Das Initiatorreagenz enthielt einen Zusatz von 10 mg/mL H-Gly-Pro-Arg-Pro-NH₂ als Fibrinaggregationshemmer. Nach 120 Sekunden Inkubation bei +37 °C wurden 25 µL Plasmin (2,23 µg/mL) zur geronnenen Probe gegeben, und nach 120, 180 und 300 Sekunden weiterer Inkubation bei +37 °C wurde der Reaktionsansatz mit gleichem Volumen Methanol gestoppt. Anschließend wurde der Reaktionsansatz 10 Minuten auf Eis gelagert und bei 13.000 UPM für ≥ 60 Sekunden zentrifugiert. 10 - 100 µL des Reaktionsansatzes wurden mittels Autosampler in das LC ESI-MS injiziert und unter folgenden Bedingungen quantifiziert:

### Flüssigkeitschromatographische Bedingungen

Die Analyse der Proben erfolgte auf einem Agilent 1100 HPLC System und einem gekoppelten LTQ-FT Massenspektrometer von Thermofisher Scientific. Die chromatographische Auftrennung der Proben erfolgte mittels 125-2- Nucleodur® C18ec, 3 µm Reverse Phase Säule von Macherey & Nagel, bei einer Säulentemperatur von 40 °C und einer Flussrate von 0,2 mL/min. Die mobile Phase bestand aus: Laufmittel A: Wasser mit 0,05% Ameisensäure und Laufmittel B: Acetonitril mit 0,045% Ameisensäure. Der Gradient war wie folgt: 0 min, 5% B; 7 min, 25% B; 15 min, 95% B; 16 min, 95% B; 17,0 min, 5% B; 25,0 min, 5% B. Die UV-Detektion erfolgte bei 215 nm.

### Massenspektrometrische Bedingungen

Für die Detektion von Bß₁₅₋₄₂-Peptid in den mittels Flüssigkeitschromatographie getrennten Reaktionsansätzen wurde ein LTQ-FT-Massenspektrometer von Thermo Fisher Scientific Corporation verwendet. Die Ionisierung der Proben erfolgte mittels Elektrospray-Ionisation (ESI) im positiven Ionisationsmodus. Die Proben wurden im Fouriertransform-MS (FTMS) pESI Full-MS-Modus im Massenbereich m/z 200 bis 2.000 auf Bß₁₅₋₄₂-Peptid unter den folgenden für das Peptid mit der Sequenz der SEQ ID NO: 9 berechneten Masse-zu-Ladung Verhältnissen der Elektrospray-Ionisations-Serie mit einer Auflösung R = 100.000 gescannt. Der für Bß₁₅₋₄₂-Peptid charakteristische Ionenpeak wurde nach ca. 5,5 Minuten Retentionszeit gemessen. Unter der hier beschriebenen massenspektrometrischen Methode zeigte Bß₁₅₋₄₂-Peptid einen dominierenden Ionenpeak bei m/z = 1013,5518 (dreifach protoniert) und 760,4158 (vierfach protoniert).Das berechnete Masse-zu-Ladung-Verhältnis (m/z) der positiven ESI-Serie für das Bß₁₅₋₄₂-Peptid ist wie folgt:
Bß₁₅₋₄₂-Peptid, SEQ ID NO: 9 (GHRPLDKKREEAPSLRPAPPPISGGGYR)

| Ladung | m/z |
|---|---|
| +1 | 3038,6399 |
| +2 | 1519,8238 |
| +3 | 1013,5518 |
| +4 | 760,4158 |
| +5 | 608,5342 |
| +6 | 507,2798 |

Tabelle 4 zeigt die Bildung des massenspektrometrisch bestimmten Fibrinspaltproduktes Bβ₁₅₋₄₂-Peptid (integrierte Peakfläche [MA] von m/z = 1013,5518) in einer Innovin®-aktivierten Plasmaprobe in Abhängigkeit von der Plasminkonzentration nach 60 Sekunden Inkubation bei +37 °C.

**Tabelle 4: Bildung von Fibrinspaltprodukt Bβ₁₅₋₄₂-Peptid in Abhängigkeit von der Plasminkonzentration**

| **Plasmin [µg/mL]** | **Bß₁₅₋₄₂-Peptid [MA]** |
|---|---|
| 1000 | 432712 |
| 400 | 215527 |
| 200 | 99542 |
| 100 | 42218 |
| 50 | 16891 |
| 0 | 4578 |

### BEISPIEL 4: Simultane, massenspektrometrische Bestimmung von Fibrinopeptid A, Fibrinopeptid B, Faktor XIII-Aktivierungspeptid und Bß15-42 Peptid in einer aktivierten Plasmaprobe

In einem Reaktionsgefäß wurden 50 µL Citratplasma mit 50 µL Innovin®-Reagenz (Siemens) oder als negative Kontrolle Wasser als Initiatorreagenz zugesetzt. Das Initiatorreagenz enthielt einen Zusatz von 2 mg/mL H-Gly-Pro-Arg-Pro-NH2 als Fibrinaggregationshemmer. Nach 0, 120 und 300 Sekunden Inkubation bei +37 °C wurde der mit Innovin® aktivierte Reaktionsansatz mit 25 µL Trichloressigsäure (TCA, 50 %ig) oder 100 µL Methanol gestoppt und nach ca. 1 Min. bei 13.000 UPM für 60 Sekunden zentrifugiert. 10 - 100 µL des gestoppten Reaktionsansatzes wurde mittels Autosampler in die LC ESI-MS injiziert und unter folgenden Bedingungen gemessen:

### Flüssigkeitschromatographische Bedingungen

Die Analyse der Proben erfolgte auf einem Agilent 1100 HPLC System und einem gekoppelten LTQ-FT Massenspektrometer von Thermofisher Scientific. Die chromatographische Auftrennung der Proben erfolgte mittels 125-2- Nucleodur® C18ec, 3 µm Reverse Phase Säule von Macherey & Nagel bei einer Säulentemperatur von 40 °C und einer Flussrate von 0,2 mL/min. Die mobile Phase bestand aus: Laufmittel A: Wasser mit 0,05% Ameisensäure und Laufmittel B: Acetonitril mit 0,045% Ameisensäure. Der Gradient war wie folgt: 0 min, 5% B; 7,0 min, 25% B; 15,0 min, 95% B; 16,0 min, 95% B; 17,0 min, 5% B; 25,0 min, 5% B. Die UV-Detektion erfolgte bei 215 nm.

### Massenspektrometrische Bedingungen

Die Ionisierung der Proben erfolgte mittels Elektrospray-Ionisation (ESI) im positiven Ionisationsmodus. Die Proben wurden im FTMS pESI Full MS Mode auf FPA und FPB wie in Beispiel 1 beschrieben und auf Bß₁₅₋₄₂-Peptid wie in Beispiel 3 beschrieben im Massenbereich m/z 300 bis 2.000, gleichzeitig mit Faktor XIII-Aktivierungspeptid (F XIII-AP), mit einer Auflösung R = 100.000 gescannt. F XIII-AP wurde unter den folgenden für die Peptide mit den Sequenzen SEQ ID Nos: 6 und 7 berechneten Masse-zu-Ladung-Verhältnissen der Elektrospray-Ionisations-Serie gescannt.

Die berechneten Masse-zu-Ladung-Verhältnisse (m/z) der positiven ESI-Serie für das humane F XIII-AP und seine V34L-Variante sind wie folgt:
F XIII-AP, SEQ ID NO: 6
(SETSRTAFGGRRAVPPNNSNAAEDDLPTVELQGVVPR)

| Ladung | m/z |
|---|---|
| +1 | 3949,9755 |
| +2 | 1975,4916 |
| +3 | 1317,3304 |
| +4 | 988,2497 |
| +5 | 790,8013 |
| +6 | 659,1691 |
| +7 | 565,1460 |

F XIII-AP (V34L), SEQ ID NO: 7
(SETSRTAFGGRRAVPPNNSNAAEDDLPTVELQGLVPR)

| Ladung | m/z |
|---|---|
| +1 | 3963,9912 |
| +2 | 1982,4995 |
| +3 | 1322,0022 |
| +4 | 991,7536 |
| +5 | 793,6045 |
| +6 | 661,5050 |
| +7 | 567,1483 |

Mit der hier beschriebenen massenspektrometrischen Methode zeigte F XIII-AP einen dominierenden Ionenpeak bei m/z = 988,2497 bzw. 991,7536 (vierfach protoniert). Die in Beispiel 1 beschriebenen massenspektrometrischen Bedingungen wurden verwendet.

Tabelle 5 zeigt die gemessene integrierte Peakfläche [MA] im FTMS pESI Full-MS-Modus von FPA (m/z 768,85), FPB (m/z 776,84), Bß₁₅₋₄₂ (m/z 1013,5518) und F XIII-AP (m/z 988,25) einer Plasmaprobe, die 0, 120 und 300 Sekunden nach Initiierung der Gerinnung mit Innovin® mittels Methanol abgestoppt wurde (Probenvolumen: 50 µL). F XIII-AP und Bß₁₅₄₂-Peptid waren bei Initiierung der Probe (0 Sek.) und Bβ₁₅₋₄₂-Peptid auch nach 120 Sekunden nicht nachweisbar (N.D.).

**Tabelle 5: Kinetik der simultanen Messung von FPA, FPB, Bß₁₅₄₂-Peptid und F XIII-AP**

| Aktivierungsdauer | FPA [MA] | FPB [MA] | F. XIII-AP [MA] | Bß₁₅₋₄₂ Peptid [MA] |
|---|---|---|---|---|
| 0 Sek. | 10213 | 9574 | N.D. | N.D. |
| 120 Sek | 4634425 | 1207136 | 9097 | N.D. |
| 300 Sek. | 14729709 | 9029314 | 90897 | 33930 |

### BEISPIEL 5: Simultane, massenspektrometrische Quantifizierung von Fibrinopeptid A, Fibrinopeptid B, Faktor XIII- Aktivierungspeptid, Dabigatran und Rivaroxaban in aktivierten Plasmaproben

Zur Bestimmung der Nachweisempfindlichkeit der Methode für Dabigatran und Rivaroxaban in Plasma wurden Dabigatran (chemische Formel: C₂₅H₂₅N₇O₃, molare Masse: 471,51) und Rivaroxaban (chemische Formel: C₁₉H₁₈ClN₃O₅S, molare Masse: 435,88) auf 15, 65, 31, 25, 62,5, 125 und 250 ng/mL in Citratplasma verdünnt. In einem Reaktionsgefäß wurden jeweils 25 µL dieser Dabigatran- und Rivaroxaban-Verdünnungsreihe in Citratplasma mit 25 µL Innovin® Reagenz oder als negative Kontrolle mit 25 µL Wasser als Initiatorreagenz aktiviert. Das Initiatorreagenz enthielt einen Zusatz von 2 mg/mL H-Gly-Pro-Arg-Pro-NH₂ als Fibrinaggregationshemmer.

Nach 0, 20 und 60 Sekunden Inkubation bei +37 °C wurde die mit Innovin® aktivierte Probe mit 100 µL Methanol (auf Eis gekühlt) gestoppt. Nach ca. 1 Min. wurden die Reaktionsansätze bei 13.000 UPM für 60 Sekunden zentrifugiert. 10 - 100 µL der gestoppten Reaktionsansätze wurden mittels Autosampler in die LC ESI-MS injiziert und unter folgenden Bedingungen gemessen:

### Flüssigkeitschromatographische Bedingungen

Die Analyse der Proben erfolgte auf einem Agilent 1100 HPLC System und einem gekoppelten LTQ-FT Massenspektrometer von Thermofisher Scientific. Die chromatographische Auftrennung der Proben erfolgte mittels Nucleodur® EC50/3-125-2-C18ec, 3 µm Reverse Phase Säule von Macherey & Nagel bei einer Säulentemperatur von 40 °C und einer Flussrate von 0,2 mL/min. Die mobile Phase bestand aus: Laufmittel A: Wasser mit 0,1 % Ameisensäure und Laufmittel B: Acetonitril mit 0,1 % Ameisensäure. Der Gradient war wie folgt: 0 min, 5% B; 4 min, 95% B; 8 min: 95% B; ab 8,2 min initiale Bedingungen bis 15 min. Die UV-Detektion erfolgte bei 215 nm.

### Massenspektrometrische Bedingungen

Die Ionisierung der Proben erfolgte mittels Elektrospray-Ionisation (ESI) im positiven Ionisationsmodus. Die Proben wurden im Fouriertransform-MS (FTMS) pESI Full-MS-Mode auf FPA bei m/z 768,85, auf FPB bei m/z 776,84 und 785,84, auf F XIIIAP bei m/z 988,25 im Massenbereich m/z 200 bis 2.000, mit einer Auflösung R = 100.000 gescannt. Für die quantitative Analyse der Antikoagulanzien wurden für Rivaroxaban eine Vorläuferionenmasse [M+H]⁺ von m/z 436,072 selektiert und Fragmentionen bei m/z 144,90 und 231,1, im ESI "ION-TRAP"-Tandemmassenspektrum im "Selected Reaction Monitoring"-Modus (pESI ITMS2 SRM) detektiert. Für Dabigatran erfolgte ein Fragmentionenscan bei m/z = 324,2 und 289,1 von der Vorläuferionenmasse (m/z) 472,19. Die in Beispiel 1 beschriebenen massenspektrometrischen Bedingungen wurden verwendet.

Tabelle 6 zeigt die gemessene integrierte Peakfläche [MA] im FTMS pESI Full-MS-Modus von FPA (m/z 768,85), FPB (m/z 776,84), F XIIIAP (m/z 988,25) sowie im pESI ITMS2 SRM-Modus von Dabigatran (Fragmentionenscan bei m/z 324,2 von Vorläuferionenmasse m/z 472,19) und Rivaroxaban (Fragmentionenscan bei m/z 144,90 von Vorläuferionenmasse m/z 436,072) einer Probe die mit 15 bis 250 ng/mL Dabigatran und Rivaroxaban gespiked wurde. Der Reaktionsansatz wurde 60 Sekunden nach Initiierung der Gerinnung mit Innovin® mittels Methanol abgestoppt. 10-100 µL des gestoppten Reaktionsansatzes wurden mittels Autosampler in die LC ESI-MS injiziert.

**Tabelle 6: Simultane massenspektrometrische Messung von FPA, FPB, F XIII-AP, Dabigatran und Rivaroxaban**

| Rivaroxaban/Dabigatran Konzentration [ng/mL] in Citratplasma | FPA | FPB | F. XIII-AP | Dabigatran | Rivaroxaban |
|---|---|---|---|---|---|
| | | | | | |
| | [MA] | [MA] | [MA] | [MA] | [MA] |
| 250 | 201837 | - | - | 288872 | 44100 |
| 125 | 318154 | - | - | 96410 | 14280 |
| 62,5 | 397224 | - | 1742 | 53460 | 3087 |
| 31,25 | 1036197 | 43633 | 4453 | 28756 | 1820 |
| 15,65 | 2361248 | 254973 | 6428 | 9316 | 586 |
| 0 | 3734995 | 807136 | 9680 | 6038 | 259 |

Die hier beschriebene Methode zeigt eine Nachweisgrenze unter 15,65 ng/mL für Dabigatran und Rivaroxaban in Innovin®-aktiviertem Citratplasma. Die Bildung von FPA, FPB und Faktor XIII-Aktivierungspeptid verhält sich nach Aktivierung der Gerinnung umgekehrt proportional zur Menge des in der Probe enthaltenen Antikoagulanzes.

## Patentansprüche

1. Verfahren zur Bestimmung der Aktivität des extrinsischen oder des intrinsischen Blutgerinnungssystems eines Individuums umfassend die Schritte:
a) Vermischen einer Probe des Individuums und eines Aktivators des extrinsischen oder des intrinsischen Gerinnungsystems zu einem Reaktionsansatz,
b) Inkubation des Reaktionsansatzes und dann
c) Bestimmung der Menge von Fibrinopeptid A und/oder Fibrinopeptid B im Reaktionsansatz.

2. Verfahren gemäß Anspruch 1, wobei die Bestimmung der Menge von Fibrinopeptid A und/oder Fibrinopeptid B im Reaktionsansatz massenspektrometrisch erfolgt.

3. Verfahren gemäß Anspruch 2, wobei die Probe des Individuums eine Plasma- oder Vollblut-Probe, bevorzugt eine Citratplasma oder Citratvollblut-Probe ist, die weder einem Verfahren zur Entfernung von Proteinen noch einem tryptischen Verdau unterworfen wurde.

4. Verfahren gemäß Anspruch 3, wobei die Probe des Individuums ferner mit einem Fibrinpolymerisationsinhibitor vermischt wird.

5. Verfahren gemäß Anspruch 4, wobei der
Fibrinpolymerisationsinhibitor ein Oligopeptid ist, das die Polymerisation von Fibrinmonomeren, die unter der Einwirkung von Thrombin entstehen, inhibiert.

6. Verfahren gemäß Anspruch 5, wobei der
Fibrinpolymerisationsinhibitor ein Oligopeptid der allgemeinen Formel GPRP-X-NH₂ ist, wobei G für die Aminosäure Glycin, P für die Aminosäure L-Prolin, R für die Aminosäure L-Arginin und X für Alanin oder Glycin steht.

7. Verfahren gemäß einem der vorhergehenden Ansprüche, wobei die Inkubation des Reaktionsansatzes durch Zugabe eines Proteaseinhibitors, durch Zugabe von Methanol, Aceton, Acetonitril, einer Säure oder einer Lauge oder durch Erhitzen des Reaktionsansatzes auf eine Temperatur zwischen 60 und 100 °C beendet wird.

8. Verfahren gemäß Anspruch 7, wobei dem Reaktionsansatz vor der Messung in Schritt c) zusätzlich mindestens 1 Volumenteil Wasser zugegeben wird.

9. Verfahren gemäß Anspruch 7, wobei das Erhitzen des Reaktionsansatzes auf eine Temperatur zwischen 60 und 100 °C durch Zugabe von mindestens 1 Volumenteil Wasser erzielt wird, welches eine Temperatur zwischen 60 und 100 °C aufweist.

10. Verfahren gemäß einem der vorhergehenden Ansprüche, wobei der Aktivator des extrinsischen Gerinnungsystems ein Gemisch aus Gewebefaktor, Phospholipiden und Calciumchlorid oder ein Schlangengift ist.

11. Verfahren gemäß einem der vorhergehenden Ansprüche, wobei der Aktivator des intrinsischen Gerinnungsystems ausgewählt ist aus der Gruppe Phospholipide, Glas, Silica, Kaolin, Ellagsäure, Celit und Plättchenfaktor 3.

12. Verfahren gemäß einem der Ansprüche 2-11, wobei in dem Reaktionsansatz zusätzlich die Menge eines oder mehrerer therapeutischer Antikoagulanzien massenspektrometrisch bestimmt wird, vorzugsweise aus der Gruppe Hirudin, Dabigatran, Melagatran, Argatroban, Ximelagatran, Bivalirudin, Lepirudin, MCC-977, SSR-182289, TGN-255, TGN-167, ARC-183 Odiparcil, Rivaroxaban, Apixaban, Edoxaban, Otamixaban,LY 517717, YM 153, DU-176b, DX-9065a und KFA-1982, Heparin, Warfarin und Phenprocoumon.

13. Verfahren gemäß einem der Ansprüche 2-12, wobei in dem Reaktionsansatz zusätzlich die Menge mindestens eines weiteren Aktivierungspeptids des Blutgerinnungssystems massenspektrometrisch bestimmt wird, vorzugsweise aus der Gruppe Faktor XIII-Aktivierungspeptid, Faktor X-Aktivierungspeptid, Faktor IX-Aktivierungspeptid und Protein C-Aktivierungspeptid.

14. Verfahren gemäß einem der Ansprüche 2-13, wobei in dem Reaktionsansatz zusätzlich die Menge mindestens eines weiteren Fibrinogenspaltproduktes massenspektrometrisch bestimmt wird, vorzugsweise des Fibrinogenspaltproduktes Bß₁₋₄₂-Peptid.

15. Verfahren gemäß einem der Ansprüche 2-14, wobei zusätzlich die Aktivität des Fibrinolysesystems des Individuums bestimmt wird, indem in dem Reaktionsansatz zusätzlich die Menge mindestens eines Fibrinspaltproduktes massenspektrometrisch bestimmt wird, vorzugsweise des Fibrinspaltproduktes Bβ₁₅₋₄₂-Peptid.

16. Verfahren gemäß einem der Ansprüche 2-15, wobei zusätzlich die Menge mindestens eines Markers der Plättchenaktivierung massenspektrometrisch bestimmt wird, vorzugsweise einer der Marker 5-Hydroxytryptamin (Serotonin), ß-Thromboglobulin oder Plättchenfaktor 4.

17. Verfahren gemäß einem der Ansprüche 12-16, wobei die massenspektrometrische Bestimmung der Menge von Fibrinopeptid A und/oder Fibrinopeptid B und die massenspektrometrische Bestimmung der Menge eines oder mehrerer therapeutischer Antikoagulanzien und/oder der Menge mindestens eines weiteren Aktivierungspeptids des Blutgerinnungssystems und/oder der Menge eines weiteren Fibrinogenspaltproduktes und/oder eines Fibrinspaltproduktes und/oder eines Markers der Plättchenaktivierung simultan durchgeführt werden.


SEQUENCE LISTING

<110>  Siemens Healthcare Diagnostics Products GmbH

<120>  VERFAHREN ZUR BESTIMMUNG DER AKTIVITÄT DES BLUTGERINNUNGSSYSTEMS

<130>  2013P11954EP

<160>  9

<170>  PatentIn version 3.5

<210>  1
<211>  16
<212>  PRT
<213>  Homo sapiens

<400>  1

Ala Asp Ser Gly Glu Gly Asp Phe Leu Ala Glu Gly Gly Gly Val Arg
1               5                   10                  15


<210>  2
<211>  15
<212>  PRT
<213>  Homo sapiens

<400>  2

Asp Ser Gly Glu Gly Asp Phe Leu Ala Glu Gly Gly Gly Val Arg
1               5                   10                  15


<210>  3
<211>  14
<212>  PRT
<213>  Homo sapiens

<400>  3

Gln Gly Val Asn Asp Asn Glu Glu Gly Phe Phe Ser Ala Arg
1               5                   10


<210>  4
<211>  14
<212>  PRT
<213>  Homo sapiens

<400>  4


<210>  5
<211>  13
<212>  PRT
<213>  Homo sapiens

<400>  5

Gln Gly Val Asn Asp Asn Glu Glu Gly Phe Phe Ser Ala
1               5                   10

<210>  6
<211>  37
<212>  PRT
<213>  Homo sapiens

<400>  6

Ser Glu Thr Ser Arg Thr Ala Phe Gly Gly Arg Arg Ala Val Pro Pro
1               5                   10                  15

Asn Asn Ser Asn Ala Ala Glu Asp Asp Leu Pro Thr Val Glu Leu Gln
            20                  25                  30

Gly Val Val Pro Arg
        35

<210>  7
<211>  37
<212>  PRT
<213>  Homo sapiens

<400>  7

Ser Glu Thr Ser Arg Thr Ala Phe Gly Gly Arg Arg Ala Val Pro Pro
1               5                   10                  15

Asn Asn Ser Asn Ala Ala Glu Asp Asp Leu Pro Thr Val Glu Leu Gln
            20                  25                  30

Gly Leu Val Pro Arg
        35

<210>  8
<211>  42
<212>  PRT
<213>  Homo sapiens

<400>  8

Gln Gly Val Asn Asp Asn Glu Glu Gly Phe Phe Ser Ala Arg Gly His
1               5                   10                  15

Arg Pro Leu Asp Lys Lys Arg Glu Glu Ala Pro Ser Leu Arg Pro Ala
            20                  25                  30

Pro Pro Pro Ile Ser Gly Gly Gly Tyr Arg
        35                  40

<210>  9
<211>  28
<212>  PRT
<213>  Homo sapiens

<400>  9

Gly His Arg Pro Leu Asp Lys Lys Arg Glu Glu Ala Pro Ser Leu Arg
1               5                   10                  15

Pro Ala Pro Pro Pro Ile Ser Gly Gly Gly Tyr Arg
            20                  25